# EUROPEAN PATENT APPLICATION

(11) **EP 4 729 035 A2**
(43) Date of publication of application: **22.04.2026**
(21) Application number: 26158549.1
(22) Date of filing: 09.03.2022
(51) Int. Cl.: A61F 5/451

(54) **FLUID COLLECTION SYSTEM SENSING AND NOTIFICATION AND RELATED METHOD**

(30) Priority: 10.03.2021 US 202163159142 P
(62) Divisional of application: 22712214.0
(71) Applicant: Purewick Corporation, Covington, Georgia 30014-1498 (US)
(72) Inventor: FALLOWS, Eric Alan, Apex, 27502 (US); KURODA, Melody Mei Hee, Durham, 27703 (US); PORTANTE, Eric, Wake Forest, 27587 (US); PATEL, Hersh, Atlanta, 30316 (US); MELLER, Camie L., Covington, 30014 (US)
(74) Representative: Studio Torta S.p.A.

(57) **Abstract**

Examples relate to devices, systems, and methods for collecting discharged fluids. A fluid collection system includes a fluid collection device configured to collect fluid discharged from a user and a fluid collection container configured to receive the fluid from the fluid collection device. The fluid collection system also includes a means for sensing at least one of a property or status of the fluid collection system. The fluid collection system also includes a controller operatively coupled to the sensor, the controller configured to communicate at least one of the property or the status of the fluid collection system to a computing device.

## Description

### CROSS-REFERENCE TO RELATED APPLICATIONS

This application claims priority to U.S. Provisional Patent Application No. 63/159,142 filed on March 10, 2021, the disclosure of which is incorporated herein, in its entirety, by this reference.

### BACKGROUND

An individual may have limited or impaired mobility such that typical urination processes are challenging or impossible. For example, the individual may have surgery or a disability that impairs mobility. In another example, the individual may have restricted travel conditions such as those experience by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated with their use. For example, bed pans may be prone to discomfort, spills, and other hygiene issues. Urinary catheters be may be uncomfortable, painful, and may cause urinary tract infections.

Thus, users and manufacturers of fluid collection devices continue to seek new and improved devices, systems, and methods to collect bodily fluids.

### SUMMARY

Embodiments disclosed herein are related to fluid collection devices and methods of using fluid collection devices. In an embodiment, a fluid collection system may include a fluid collection device configured to collect fluid discharged from a user and a fluid collection container configured to receive the fluid from the fluid collection device. The fluid collection system also includes a means for sensing at least one of a property or status of the fluid collection system and a controller operatively coupled to the means for sensing, the controller configured to communicate at least one of the property or the status of the fluid collection system to a computing device.

**In** an embodiment, a method of using a fluid collection system may include placing a fluid collection device at least proximate to a urethra of a user and receiving fluid discharged from the user in a fluid collection device. The method further includes receiving fluid discharged from the fluid collection device in a fluid collection container. The method also includes detecting, with a means for sensing, at least at least one of a property or status of the fluid collection system. The method also includes transmitting, from a controller coupled to the means for sensing, to a computing device the at least one of the property or the status of the fluid collection system.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG.** 1A is a block diagram of a fluid collection system, according to an embodiment.
**FIG. 1B** is an isometric view of a flow meter, according to an embodiment.
**FIG.** 1C is a schematic view of the flow meter of FIG. 1B, with a float in a visible region.
**FIG.** 1D is a schematic view of a flow meter including a spring, according to an embodiment.
**FIG.** 1E is a schematic view of the flow meter of FIG. 1B, with the float in a non-visible region.
**FIG. 2A** is a block diagram of a fluid collection system, according to an embodiment.
**FIG. 2B** is an isomeric view of a fluid collection system, according to an embodiment.
**FIG. 2C** is a cross sectional view of a fluid collection container, according to an embodiment.
**FIG.** 3 is a schematic for a computing device in a controller of a fluid collection system, according to an embodiment.
**FIG. 4A** is a schematic view of a module with a display indicating a property or status of the fluid collection system, according to an embodiment.
**FIG. 4B** is a schematic view of a smartphone with a display indicating a property or status of the fluid collection system, according to an embodiment.
**FIG.** 5 is a flow diagram of a method of using a fluid collection system, according to an embodiment.

### DETAILED DESCRIPTION

Embodiments disclosed herein are related to fluid collection systems including a means for sensing at least one of a property or status of the fluid collection system, and methods of using the same. The devices and systems disclosed herein are configured to collect fluids from an individual and include at least one sensor. The fluids collected by the fluid collection devices may include at least one of urine, vaginal discharge, penile discharge, reproductive fluids, blood, sweat, wound discharge, interstitial fluid, cerebrospinal fluid, or other bodily fluids. Embodiments of fluid collection systems disclosed herein may include a means for sensing at least one of a property or status of the fluid collection system and a controller operatively coupled to the means for sensing, the controller configured to communicate at least one of the property or the status of the fluid collection system to a computing device. The at least one property of the fluid collection system may be related at least to a volume of the fluid exiting a fluid collection device and/or entering a fluid collection container.

In some embodiments, the property may include at least one of a volume, a mass, or a velocity of the fluid passing through or proximate a tube in fluid communication with the fluid collection device. For example, the means for sensing may include a flow meter coupled to the tube that may be configured to measure a volumetric flow rate of the fluid. In some embodiments, the flow property may include a flow rate of the fluid over a predetermined period of time. At least one of the means for sensing may include a sensor disposed within an interior portion of the fluid collection container, wherein the sensor is configured to detect a property related to the volume or mass of the fluid in the fluid collection container.

The fluid collection system also may include a controller operatively coupled to the means for sensing, the controller configured to communicate at least one of the property or the status of the fluid collection system to a computing device. By sensing at least one of a property or status of the fluid collection system and having a controller that communicates at least one of the property or status of the fluid collection system to the computing device, more accurate volumes, volumetric flow rates, and fluid collection system information and/or status may be determined, collected, and/or utilized in real-time. The greater accuracy and convenience provided to the user and/or caretaker may result in a cleaner and more sanitary fluid collection system and optimization of care.

In some examples, the computing device or system receiving at least one of the detected flow property or the volume of the fluid may include a module attached to an arm of a wheelchair or a smartphone. As shall be described in greater herein, the computing device may include a programmable timer, an alert or alarm, or a display configured to display the at least one property of the fluid or the status of the fluid collection system. In some examples, the computing device may be wirelessly coupled to the controller.

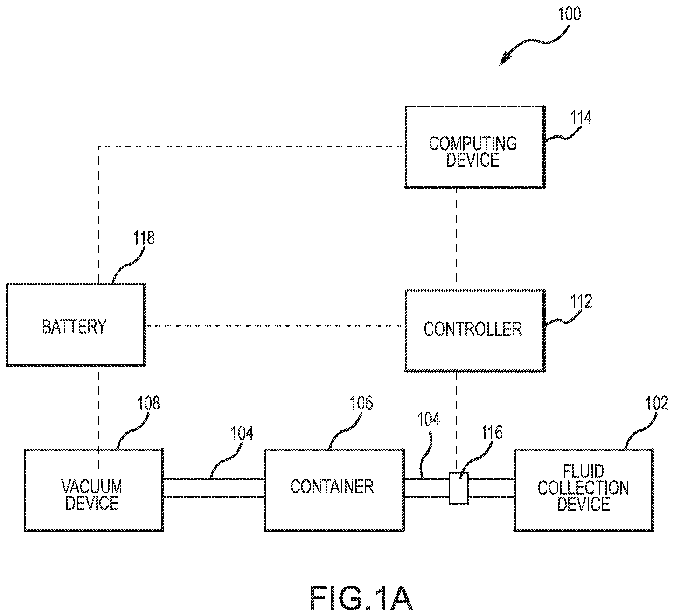

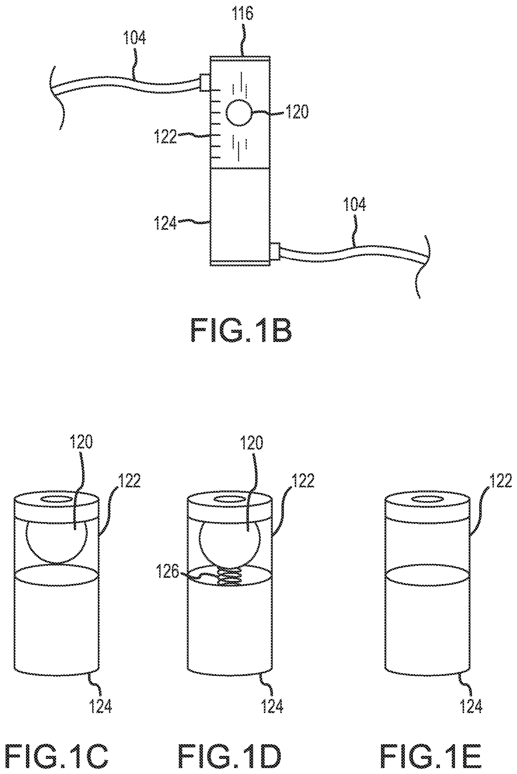

**FIG.** 1A is a block diagram of a fluid collection system 100, according to an embodiment. The fluid collection system 100 includes a fluid *(e.g.,* urine) collection device 102 configured to collect fluid discharged from a user. The fluid collection device 102 may include a male or female collection device. PCT International Application No. PCT/US2019/029616, for example, describes various embodiments of both male and female fluid collection devices, the disclosure of which is incorporated, in its entirety, by this reference. Moreover, the fluid collection device 102 may be interchangeable in the fluid collection system 100 between different types, varieties, and sizes of male or female fluid collection devices. The fluid collection device 102 may be configured to be positioned proximate or adjacent to a urethra of a user. Generally, the fluid collection device 102 may include a surface sized to be positioned proximate or adjacent to the urethra and configured to wick fluid or other fluids away from the user. Urine or other fluids may be wicked from the surface to a reservoir in the fluid collection device 102.

**In** some embodiments, the fluid collection system 100 may include a tube 104 that couples the fluid collection device 102 to a fluid collection container 106 and a vacuum device or pump 108. The tube 104 may include a flexible material such as plastic tubing *(e.g.,* medical tubing). Such plastic tubing may include a thermoplastic elastomer, polyvinyl chloride, ethylene vinyl acetate, polytetrafluoroethylene, etc., tubing. In some embodiments, the tube 104 may include silicone or latex. In other embodiments, the tube 104 may include a reinforced tube having metal or other electrically conductive components therein. The fluid collection device 102, the container 106, and the pump 108 may be fluidly coupled to each other via one or more tubes 104. For example, fluid collection device 102 may be operably coupled to one or more of the fluid collection container 106 or the pump 108 via the tube 104. Fluid *(e.g.,* urine or other bodily fluids) collected in the fluid collection device 102 may be removed from the fluid collection device 102 via the tube 104 coupled to the fluid collection device 102. A vacuum may be introduced into an interior chamber of the fluid collection device 102 via an inlet of the tube 104 responsive to suction (e.g., vacuum) force applied at an outlet of the tube 104.

The vacuum or suction force may be applied to the tube 104 by the pump 108 either directly or indirectly. The vacuum may be applied indirectly via the fluid collection container 106. For example, the outlet of the tube 104 may be connected to or disposed within the fluid collection container 106 and an additional tube 104 may extend from the fluid collection container 106 to the pump 108 or other suitable vacuum device. Accordingly, the pump 108 may apply a suction to the fluid collection device 102 via the fluid collection container 106. The vacuum may be applied directly via the pump 108. For example, the outlet of the tube 104 may be disposed within the pump 108. An additional tube 104 may extend from the pump 108 to a point outside of the fluid collection device 102, such as to the fluid collection container 106. In other embodiments, the pump 108 may be coupled to an exterior of the tube 104 to draw vacuum, such as a peristaltic pump. In such examples, the pump 108 or other vacuum device may be disposed between the fluid collection device 102 and the fluid collection container 106.

The pump 108 may include one or more of a manual vacuum pump, and electric vacuum pump, a diaphragm pump, a centrifugal pump, a displacement pump, a magnetically driven pump, a peristaltic pump, or any pump configured to produce a vacuum. The pump 108 may provide a vacuum or suction to remove fluid from the fluid collection device 102 that may be discharged from the user. In some examples, the pump 108 may be powered by one or more of a power cord *(e.g.,* connected to a power socket), one or more batteries, or even manual power *(e.g.,* a hand operated vacuum pump). In some examples, the pump 108 may be sized and shaped to fit outside of, on, or within the fluid collection device 102. For example, the pump 108 may include one or more miniaturized pumps or one or more micro pumps.

The fluid collection container 106 may be sized and shaped to retain a fluid therein. The fluid collection container 106 may include a bag (e.g., drainage bag), a bottle or cup *(e.g.,* collection jar), or any other enclosed container for storing bodily fluid(s) such as urine. In some embodiments, the fluid collection container 106 may include a generally rigid exterior housing an interior portion configured to contain the fluid. In some examples, the tube 104 may extend from the fluid collection device 102 and attach to the fluid collection container 106 at a first point therein. An additional tube 104 may couple to the fluid collection container 106 at a second point therein and may extend and attach to the pump 108. Accordingly, the pump 108 may be configured to pull an at least partial vacuum to draw the fluid from the fluid collection device 102 through the tube 104 into the fluid collection container 106. Fluid, such as urine, may be drained from the fluid collection device 102 and into the fluid collection container 106 via the pump 108.

The fluid collection system 100 may include a means for sensing 110 at least one of a property or status of the fluid collection system 100. The means for sensing 110 may include at least one sensor, flow meter, and/or device to obtain the status of the fluid collection system 100 or a fluid property within the fluid collection system 100. In some embodiments, the means for sensing 110 may include a flow meter 116 coupled to the tube 104 and configured to detect a vacuum status in the tube 104 or measure a volumetric flow rate of the fluid. In some embodiments, metal or other electrically conductive components within the tube 104 may act as an electromagnetic flowmeter (not shown). The electromagnetic flowmeter may include a conductance or capacitance change as a means for sensing flow. The fluid collection system 100 may also include a controller 112 operatively coupled to the flow meter such that the flow meter 116 coupled with the controller are the means for sensing 110, the controller configured to communicate at least one of the property or status of the fluid collection system 100 to a computing device 114.

The flow meter 116 also may include or be operably coupled to a controller 112 operatively coupled to the flow meter 116, the controller configured to communicate the at least one of the flow property or status of the fluid to the computing device 114. In some embodiments, the controller 112 may be separate or external to the flow meter 116. For example, the flow meter 116 may be configured to wirelessly communicate via BLUETOOTH or WI-FI to the computing device 114 data including at least one of the detected flow properties or the volume of the fluid discharged from the user and entering the fluid collection container 106. In some embodiments, the controller 112 may be integrated with the computing device 114. In some embodiments, the flow meter 116 may be configured to wirelessly or communicate the volumetric flow data to the computing device 114 in predetermined intervals or whenever fluid flow is detected. The computing device 114 may include various computing devices configured to wirelessly communicate with the flow meter 116. For example, the computing device 114 may include a module coupled to a wheelchair or bed, a smartphone, a smart wearable (e.g., wrist device), a tablet computer, a desktop computer, or medical computing devices found in hospital or healthcare rooms.

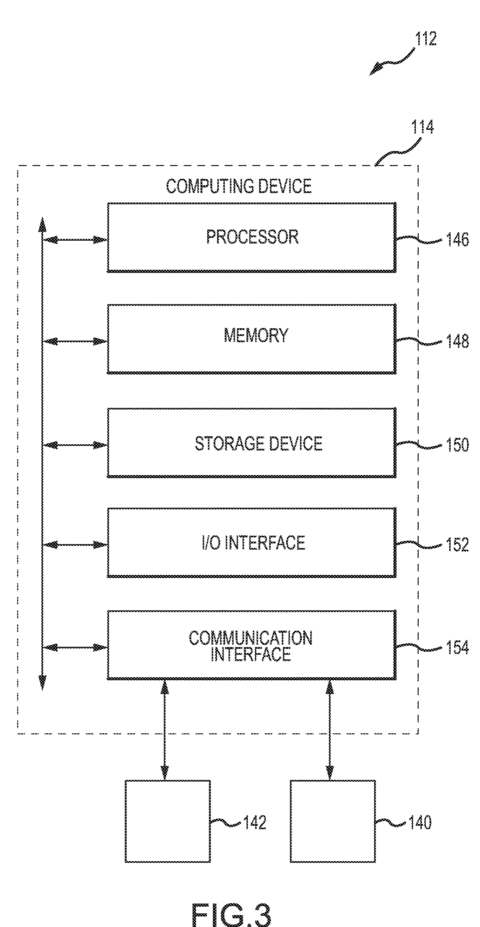

In some embodiments, the flow meter 116 may be configured to wirelessly communicate the flow property of the fluid to the controller 112 which may then wirelessly communicate the volumetric flow rate to the computing device 114 which may include a software application configured to record data including an amount of fluid received or drank by the user, the flow rate of the fluid, display the flow rate and/or volume of the fluid discharged by the user, and/or determine and display trends and totals related to the flow rate and/or volume of the fluid discharged by the user. The computing device 114 may be located on the user, coupled to a wheelchair, in a hospital room, in a home of the user, or combinations thereof. In some examples, the pump 108, the means for sensing 110, the controller 112, and the computing device 114 may be powered by one or more batteries 118. Additional details regarding the schematics, power supply, and wireless communications of the controller 112 and the computing device 114 are provided below in reference to **FIG. 3****.**

Referring now to **FIGS. 1B-1E****,** in some embodiments, the means for sensing 110 includes a flow meter 116 configured to detect a detect a flow property of the fluid at least related to a volume of the fluid pulled from the fluid collection device 102 and passing through tube 104 before entering the fluid collection container 106. In some embodiments, the flow meter 116 may be coupled to the tube 104 between fluid collection device 102 and the fluid collection container 106 such that a first portion of the tube 104 extends between the fluid collection device 102 and the flow meter 116 and a second portion of the tube 104 extends between the flow meter 116 and the fluid collection container 106. In some embodiments, the flow meter 116 may be coupled to the fluid collection device 102. In some embodiments, the flow meter 116 may be coupled to the fluid collection container 106. The flow meter 116 may include any suitable flow meter configured to detect a flow property of the fluid that may be used to determine a presence of the vacuum or volume of the fluid passing through or within a predetermined proximity to the flow meter 116.

**In** some embodiments, the flow meter 116 may include an in-line ball style sight flow indicator disposed within the tube 104. The ball style sight flow indicator may include a ball or other suitable shaped float 120. In some embodiments, the float 120 may be visible when the vacuum is drawn and not visible when the vacuum is absent. In some embodiments the flow meter 116 may include a visible region 122 and a non-visible region 124. The visible region 122 may include a transparent viewing window. The viewing window may include markings to indicate a flow rate within the flow meter 116 or tube 104. The non-visible region 124 may hide the float 120 when the vacuum is absent. The float 120 in the non-visible region 124 may indicate at least one status of the fluid collection system 100. In some embodiments, when the float 120 is in the non-visible region 124, the pump 108 may be off or inoperable. In other embodiments, the tube 104 may be clogged, kinked, or disconnected. In yet other embodiments, the fluid collection container 106 may be full or overflowing, or the fluid collection system 100 may have a leak or otherwise failing component. In some embodiments, the float 120 may be supported by the stream of fluid in the tube 104. The float 120 may be a ball and/or fluted to assist the float 120 to remain in the center of the fluid flow.

In some embodiments, the float 120 may be coupled to a spring 126 or other suitable compliant member or mechanism. The flow meter 116 may include the spring 126 and a piston *(e.g.,* ball float 120) flow indicator disposed within the first tube 104, wherein the spring 126 may be calibrated and holds the piston such that the piston is visible when the vacuum is drawn and not visible when the vacuum is absent. As discussed above, the flow meter 116 may include a visible region 122 and a non-visible region 124 and the piston may operate in a similar manner as the float 120 described previously.

In some embodiments, the flow property detected by the flow meter 116 may include at least one of a volume, a mass, or a velocity of the fluid passing through or proximate to the flow meter 116. The flow meter 116 may include a positive displacement flow meter configured to directly measure the actual volume of the fluid passing through the flow meter 116. In some embodiments, the flow meter 116 is configured to detect a mass or a velocity of the fluid passing through or proximate to the flow meter 116, which mass or velocity may be translated (by at least one of the flow meter 116 or a computing device) into a volume or flow rate. The flow meter 116 may detect a duration of time during which fluid is passing through or within a predetermined proximity to the flow meter 116. The flow meter 116 or other computing device may then calculate that volume of the fluid that has passed the flow meter 116 based on a known cross-sectional area or volume of the tube 104 or the flow meter 116.

In some embodiments, the flow meter 116 may mechanically or ultrasonically detect the flow property of the fluid passing between the fluid collection device 102 and the fluid collection container 106. For example, at least a portion of the flow meter 116 may be in line with the tube 104 effective to allow the flow meter 116 to mechanically detect the flow property of the fluid through the tube 104. In some embodiments, the flow meter 116 may be incorporated into the tube 104. In some embodiments, at least a portion of the flow meter 116 extends into the tube 104 effective to allow the flow meter 116 to mechanically detect the flow property of the fluid through the tube 104. The flow meter 116 may include at least one of a bi-rotor, a disc, a piston, a propeller, a paddle wheel, and/or a rotameter. The flow meter 116 may include laminar or venturi flow meter. In some embodiments, the flow meter 116 may be detachably or fixedly secured to the outside of the tube 104 and ultrasonically *(i.e.,* via the Doppler effect) or electromagnetically detect the flow property of the fluid through the tube 104.

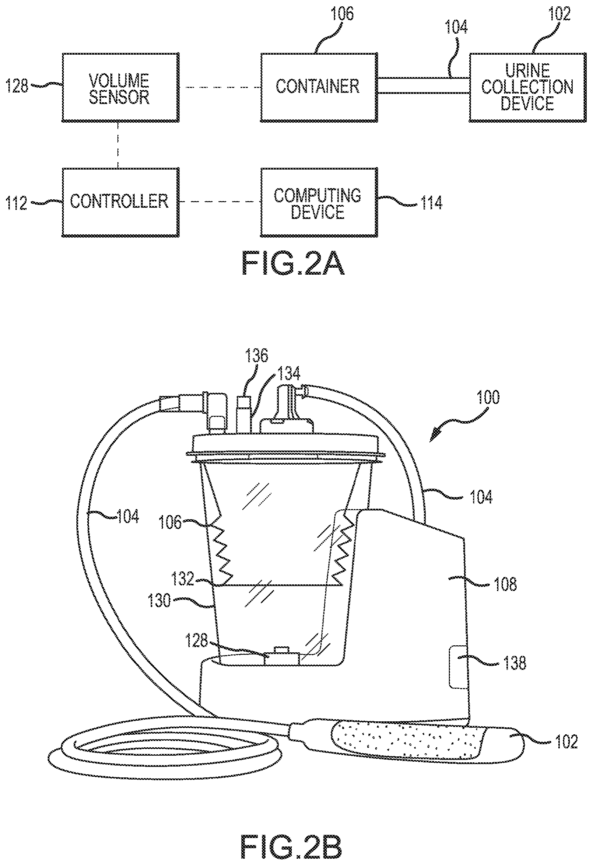

Referring now to **FIG. 2A****,** the means for sensing 110 of the fluid collection system 100 also may include a volume sensor 128. The volume sensor 128 may be coupled to the fluid collection container 106 and configured to detect a property relating at least to a volume of the fluid in an interior portion of the fluid collection container 106. In some embodiments, the fluid collection container 106 may include an exterior housing 130 and an interior portion 132. The interior portion 132 may be configured to contain the fluid. In some embodiments, the volume sensor 128 may be disposed between the exterior housing 130 and the interior portion 132. In other embodiments, the volume sensor 128 may be located or positioned at an inlet (either interior or exterior) of the fluid collection container 106 for the tube 104. The volume sensor 128 may be positioned along and/or within the tube 104, according to an embodiment.

Similar to the flow meter 116 above, the volume sensor 128 may also include a controller 112 operatively coupled to the volume sensor 128, the controller 112 configured to communicate the volume of fluid in the fluid collection container 106 to the computing device 114. The controller 112 may be configured such that the volume sensor 128 and the controller are the means for sensing 110 that communicates at least one of the property or status of the fluid collection system 100 to a computing device 114. In some embodiments, the controller 112 may be separate or external to the volume sensor 128 and/or the fluid collection container 106. The controller 112 may be internal or coupled to the computing device 114. The computing device 114 may be the same computing device 114 discussed above and/or may include various computing devices configured to wirelessly communicate with the volume sensor 128. For example, the computing device 114 may include a module coupled to a wheelchair or bed and/or a smartphone.

Referring now to **FIG. 2B****,** in some embodiments, the exterior housing 130 may be opaque or clear according to different embodiments and may include a generally rectangular front or rear profile. In some embodiments, the exterior housing may include at least one of a handle or a spout 134. Fluid collected in the interior portion 132 may be emptied through the spout 134 after removing a cap or cover 136. The exterior housing 130 may be reusable and dishwasher safe, and may include a generally rigid material such as polycarbonate, plastic, rubber, metal, glass, combinations thereof, or any other suitable materials. In some embodiments, the interior portion 132 may be expandable. The interior portion 132 may include a bellows shape that expands as it fills with the fluid. In some embodiments, the interior portion 132 may be an expandable bag. For example, the interior portion 132 may be generally flat when empty, but may expand when urine or other fluid is forced into the interior portion 132. In other embodiments, the interior portion 132 may include a material that expands when wetted. In some embodiments, the interior portion 132 may be constructed of polycarbonate, plastic, rubber, metal, latex. In other embodiments, the interior portion may be hydroscopic, such as paper, cotton, wood, nylon, super absorbent polymer (SAP) such as sodium polyacrylate, polyacrylamide, or other suitable material. In some embodiments, the volume sensor 128 may be configured to activate when the interior portion 132 expands to a predetermined volume. In some embodiments, the volume sensor 128 may include a switch located on an interior surface of the exterior housing 130 that activates when the interior portion 132 expands to compress the switch between the interior portion 132 and the exterior housing 130. In other embodiments, the volume sensor 128 may be located on a side wall of the exterior housing 130, the exterior surface of the interior portion 132, or any suitable location between the exterior housing 130 and the interior portion 132.

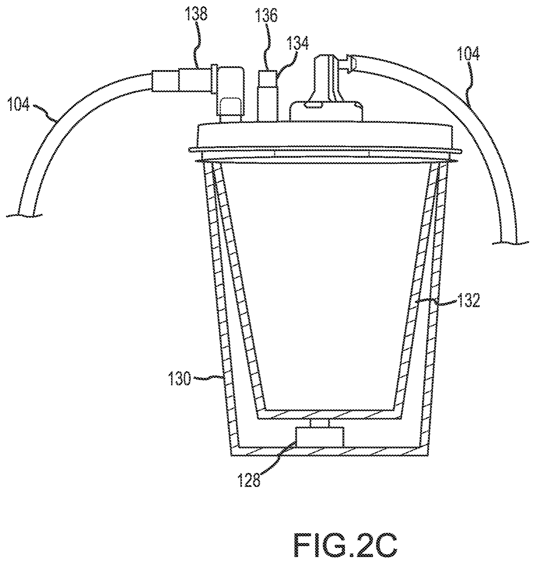

Referring now to **FIG. 2C****,** in some embodiments, the interior portion 132 may be generally rigid and the volume sensor 128 may include at least one of a pressure sensor, a capacitive sensor, or an optical sensor. In some embodiments, the pressure sensor may be configured operate an electrical contact when a predetermined fluid mass is reached. The pressure sensor may close an electrical contact or communicate with the controller 112 on the pressure increase caused by the interior portion 132 being filled with fluid. The capacitive sensor may include a proximity sensor or non-contact sensor that may be configured to utilize the electrical property of capacitance and the change of capacitance based on a change in the electrical field around an active face of the capacitive sensor. In some embodiments, as the interior portion 132 approaches the exterior housing 130, a metal plate in the sensing face of the capacitive sensor may be electrically connected to an internal oscillator circuit and the surface of the interior portion to be sensed acts as the second plate of the capacitor and may be configured to operate an electrical contact and/or communicate with the controller 112 when a predetermined distance between the surfaces of the interior portion 132 and exterior housing 130 is reached. In an optical sensor, light rays may be converted into an electronic signal. In an embodiment, an optical sensor may be coupled to an electrical switch that activates and/or signals the controller 112 upon a change in the signal within the optical sensor when a surface of the interior portion 132 approaches a predetermined distance from the exterior housing 130.

In some embodiments, the means for sensing 110 may include a system integrity sensor 138. The system integrity sensor 136 may be configured to detect when the fluid collection device 102 is decoupled from the fluid collection container 106. The integrity sensor 138 may be coupled to the fluid collection device 102, the pump 108 as shown in FIG. 2B, the flow meter 116, the exterior housing 130 of the fluid collection container 106, or any suitable location or configuration to determine whether components are decoupled and/or malfunctioning. In some embodiments, the system integrity sensor 138 may include a simple contact switch between components such as between the tube 104 and the fluid collection container 106, as shown in FIG. 2C.

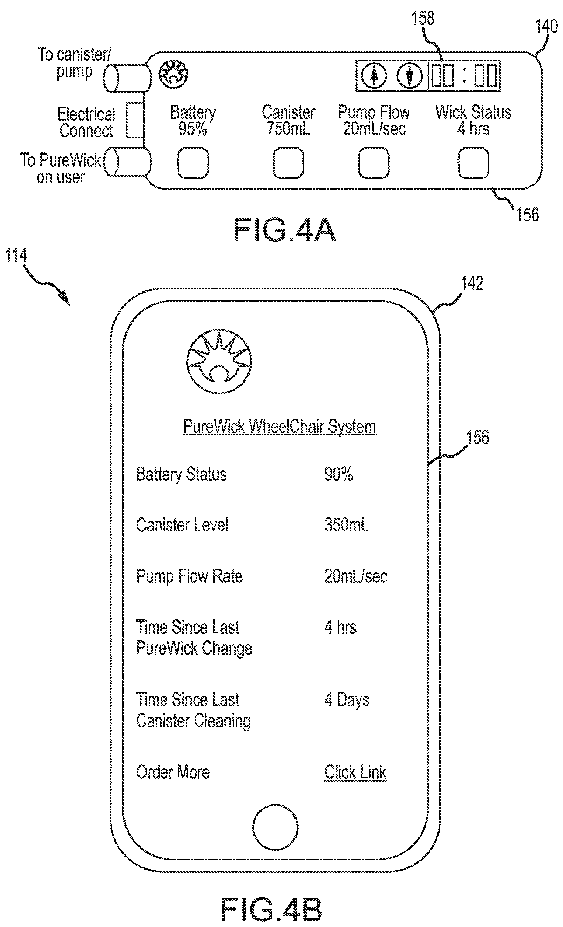

FIG. 3 is a schematic of controller 112 that may be integrated into any of the means for sensing 110 discussed above or the computing device 114 described herein, according to some embodiments. The controller 112 may be configured to communicate with any of the means for sensing 110, such as with a wired or wireless connection. In some embodiments, the means for sensing 110 may include the controller 112. In an embodiment, the controller 112 may include a printed circuit board (PCB) equipped with erasable programmable read-only memory (EPROM) for memory of at least data collected by the means for sensing 110. For example, the controller 112 may be configured to calculate a level or volume of fluid in the fluid collection container 106 or a volumetric flow rate or presence of fluid within the first tube 104. The controller 112 may be configured to send notifications or alerts to other electronic devices. For example, the controller 112 may be configured to send notifications or alerts to a module 140 (shown in FIG. 4A) attached to an arm of a wheelchair at a selected radio frequency, via BLUETOOTH, or via WI-FI to another electronic device, such as a smartphone 142 (shown in FIG. 4B) of the user or caregiver. The controller 112 may be powered by an external or internal battery, such as a rechargeable battery or the battery 118.

In some embodiments, the controller 112 may be configured to wirelessly transmit an alert to the module 140 and/or the smartphone 142 of the user or a caregiver a property relating at least to the status of the pump 108. In some embodiments, the controller 112 may be configured to operate and/or control the pump 108. In some embodiments, the controller 112 may control the pump 108 based off the status of the volume of fluid detected by the volume sensor 128 in the fluid collection container 106. For example, based on data from the means for sensing 110, the controller 112 may start the pump 108 to cause fluid to flow from the fluid collection device 102 to the fluid collection container 106. The controller 112 may cause the pump 108 to secure when the volume of fluid in the fluid collection container 106 reaches a predetermined threshold. In some embodiments, the controller 112 may wirelessly transmit alerts and selected frequencies, such as selected time and/or volume intervals. The controller 112 may wirelessly communicate to the module 140 or the smartphone 142 of the user or the caregiver when the battery 118 is low. The controller 112 may transmit an alert to the module 140 and/or the smartphone 142 of the user or the caregiver when the pump 108 or fluid collection system 100 has a malfunction. In some embodiments, the controller 112 may be configured to control all operations of the pump 108.

The controller 112 may include at least one computing device 114, according to an embodiment. The at least one computing device 114 may be an exemplary computing device that may be configured to perform one or more of the acts described above. The computing device 114 may include at least one processor 146, memory 148, a storage device 150, an input/output ("I/O") device/interface 152, and a communication interface 154.

In some examples, the processor 146 may include hardware for executing instructions (e.g., instructions for carrying out one or more portions of any of the methods disclosed herein), such as those making up a computer program. For example, to execute instructions, the processor 146 may retrieve the instructions from an internal register, an internal cache, the memory 148, or a storage device 150 and decode and execute them. In some examples, the processor 146 may be configured (e.g., include programming stored thereon or executed thereby) to carry out one or more portions of any of the example methods disclosed herein.

In some examples, the processor 146 may be configured to perform any of the acts disclosed herein or cause one or more portions of the computing device 114 or controller 112 to perform at least one of the acts disclosed herein. Such configuration can include one or more operational programs (e.g., computer program products) that are executable by the at least one processor 146. For example, the processor 146 may be configured to automatically determine a volume of urine in a urine collection container, automatically determine a proximity of urine in the urine collection container to a sensor, automatically transmit an alert when the volume of the urine in the urine collection container meets or exceeds a predetermined threshold, automatically transmit an alert when a fluid is sensed in the fluid collection device 102, determine a difference in fluid intake and fluid output of a user, and/or automatically transmit an alert when a change or recharge of battery is suggested.

The at least one computing device 114 may include at least one memory storage medium *(e.g.,* memory 148 and/or storage device 150). The computing device 114 may include memory 148, which is operably coupled to the processor 146. The memory 148 may be used for storing data, metadata, and programs for execution by the processor 146. The memory 148 may include one or more of volatile and non-volatile memories, such as Random Access Memory (RAM), Read Only Memory (ROM), a solid state disk (SSD), Flash, Phase Change Memory (PCM), or other types of data storage. The memory 148 may be internal or distributed memory.

The computing device 114 may include the storage device 150 having storage for storing data or instructions. The storage device 150 may be operably coupled to the at least one processor 146. In some examples, the storage device 150 may comprise a non-transitory memory storage medium, such as any of those described above. The storage device 150 *(e.g.,* non-transitory storage medium) may include a hard disk drive (HDD), a floppy disk drive, flash memory, an optical disc, a magneto-optical disc, magnetic tape, or a Universal Serial Bus (USB) drive or a combination of two or more of these. Storage device 150 may include removable or non-removable (or fixed) media. Storage device 150 may be internal or external to the computing device 114. In some examples, storage device 150 may include non-volatile, solid-state memory. In some examples, storage device 150 may include read-only memory (ROM). Where appropriate, this ROM may be mask programmed ROM, programmable ROM (PROM), erasable PROM (EPROM), electrically erasable PROM (EEPROM), electrically alterable ROM (EAROM), or flash memory or a combination of two or more of these. In some examples, one or more portions of the memory 148 and/or storage device 150 *(e.g.,* memory storage medium(s)) may store one or more databases thereon.

In some examples, one or more of a history of the volume of the fluid in the fluid collection container 106, a trend of the volume of the fluid in the fluid collection container 106, a history of a fluid collection device 102 replacement, and/or a history of battery 118 replacement or recharging may be stored in a memory storage medium such as one or more of the processor 146 *(e.g.,* internal cache of the processor), memory 148, or the storage device 150.

The computing device 114 also includes one or more I/O devices/interfaces 152, which are provided to allow a user to provide input to, receive output from, and otherwise transfer data to and from the computing device 114. These I/O devices/interfaces 152 may include a mouse, keypad or a keyboard, a touch screen, camera, optical scanner, network interface, web-based access, modem, a port, other known I/O devices or a combination of such I/O devices/interfaces 152. The touch screen may be activated with a stylus or a finger.

The I/O devices/interfaces 152 may include one or more devices for presenting output to a user, including, but not limited to, a graphics engine, a display *(e.g.,* a display screen or monitor), one or more output drivers (e.g., display drivers), one or more audio speakers, and one or more audio drivers. In certain examples, I/O devices/interfaces 152 are configured to provide graphical data to a display for presentation to a user. The graphical data may be representative of one or more graphical user interfaces and/or any other graphical content as may serve a particular implementation.

The computing device 114 may further include a communication interface 154. The communication interface 154 may include hardware, software, or both. The communication interface 154 may provide one or more interfaces for communication (such as, for example, packet-based communication) between the computing device 114 and one or more networks. For example, communication interface 154 may include a network interface controller (NIC) or network adapter for communicating with an Ethernet or other wire-based network or a wireless NIC (WNIC) or wireless adapter for communicating with a wireless network, such as a WI-FI. As discussed above, in some embodiments, the computing device 114 may communicate with and/or control the operations of the pump 108.

Referring now to **FIGS. 4A-4B****,** in some embodiments, the computing device 114 may communicate with a module 140 and/or smartphone 142 that may include a display 156. In an embodiment, the controller 112 may be configured to transmit a signal including at least one of a property or status of the fluid collection system 100 to the module 140 and/or the smartphone 142. In some embodiments, the display 156 may include at least one of a property or status of the fluid collection system 100. For example, the display 156 may include one or more of a battery status, a volume of fluid in the interior portion 132 of the fluid collection container 106, a volumetric flow rate, a time since last change of the fluid collection device 102, time since last cleaning of a component of the fluid collection system 100, an amount of fluid intake by the user, a difference in fluid intake by the user and the volume of fluid in the interior portion 132 of the fluid collection container 106, or a system integrity status. For example, the computing device may indicate a net fluid loss of a user by determining the difference between the volume of fluid in the interior portion 132 as indicated by the volume sensor 128 and the fluid intake. As another example, based on data from the volume sensor 128, the controller 112 may wirelessly transmit an alert to the smartphone 142 of a caregiver that the interior portion 132 of the fluid collection container 106 is at a predetermined distance (such as about 1 inch) from the volume sensor 128 and/or the exterior housing 130 and emptying of the fluid collection container 106 is recommended. In some embodiments, the controller 112 may wirelessly transmit alerts and selected frequencies, such as selected time and/or volume intervals. In some embodiments, the computing device 114 may be configured to provide the at least one of the property or status of the fluid collection system 100 in real-time as communicated by the controller 112. The controller 112 may wirelessly transmit to the computing device 114 a property or status of the fluid collection system 100 or an alert to the electronic device of the user or the caregiver when a battery powering at least one of the controller 112, the module 140, or the pump 108 is low. The controller 112 may wirelessly transmit an alert to the electronic device of the user or the caregiver when cleaning or replacement of the fluid collection device 102 or other component may be due. In some embodiments, the controller 112 may issue an alarm and/or alert to the user or caregiver. The alert may include a light, a sound, or a message on the module 140 or smartphone 142 display 156. In an embodiment, the display 156 may provide at least the current status of the battery 118, the fill level of the fluid collection container 106, an input from the user or caregiver to include the volume of fluid consumed by the user, the time since the last replacement or cleaning of the fluid collection device 102, and the time since the least cleaning or replacement of the fluid collection container 106. In an embodiment, the computing device 114 may include a programmable timer 158. For example, the timer 158 may include an input from the user or caregiver to replace a component of the fluid collection system 100, empty the fluid collection container 106, or start or stop the pump 108. While an example computing device 114 is shown in **FIGS. 4A-4B****,** the components illustrated in **FIGS. 4A-****4B** are not intended to be limiting of the controller 112 or computing device 114. Additional or alternative components may be used in some examples. In some examples, the smartphone 142 may include a hyperlink to a vendor for ordering new fluid collection devices, fluid collection containers, etc. Further, in some examples, the controller 112 or the computing device 114 may include fewer components than those shown in FIG. 4A-4B.

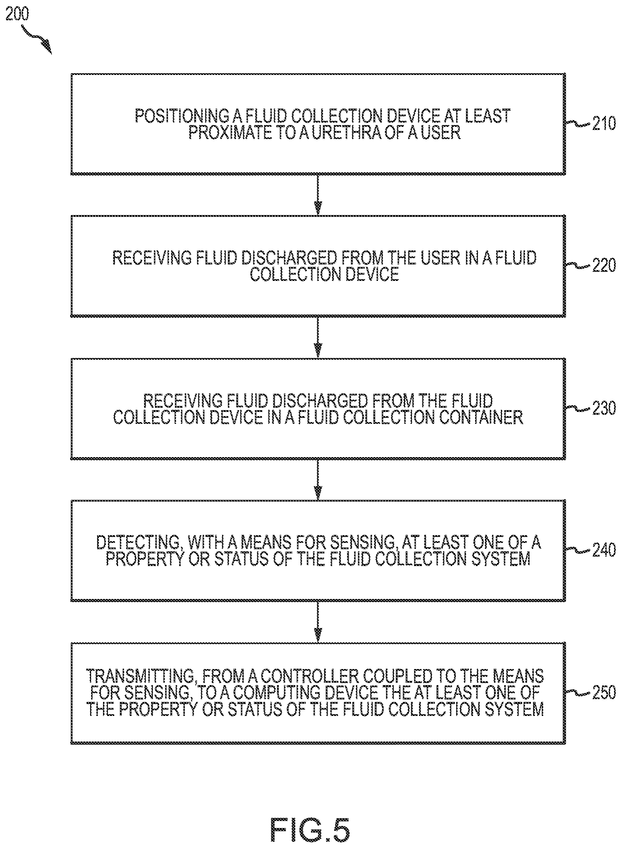

**FIG.** 5 is a flow diagram of a method 200 for assembling a portable fluid collection system, according to an embodiment. The method 200 includes an act 210 of positioning a fluid collection device at least proximate to a urethra of a user and an act 220 of receiving fluid discharged from the user in a fluid collection device. The method 200 also includes an act 230 of receiving fluid discharged from the fluid collection device in a fluid collection container. In some embodiments, the method 200 may further include using a pump to pull a vacuum on a first tube in fluid communication with the fluid collection device effective to draw fluid from the fluid collection device and into the fluid collection container.

The method 200 also includes an act 240 of detecting, with a means for sensing, at least one of a property or status of the fluid collection system. In some embodiments, the means for sensing may include a flow meter. The act 240 may include mounting detecting at least one of a volume of fluid within the fluid collection device or the fluid collection container, a flow property of the fluid passing through a first tube, a pump status, or a battery status. The method 200 also includes and act 250 of transmitting, from a controller coupled to the means for sensing, to a computing device the at least one of the property or status of the fluid collection system. In some embodiments, act 250 may include transmitting to a module attached to an arm of a wheelchair or a smartphone. In an embodiment, method 200 may also include providing, with the computing device, the at least one property or status of the fluid collection system in real-time as communicated by the controller.

The method 200 may include any of the fluid collection systems and/or devices described or incorporated by reference herein. For example, the act 210 of detachably securing a container support to the wheelchair may include securing one or more straps coupled to a cushion cover to one or more structural components of the chair, the wheelchair, or the bed. In some embodiments, the act 210 of detachably securing a fluid collection system to at least one of a chair, a wheelchair, or a bed may include positioning the fluid collection container within the cushion and placing the cushion within a cushion cover. In some embodiments, the cushion cover encloses the fluid collection pad inside a pocket. The act 210 of removing and replacing the fluid collection pad when a volume of the fluid in the fluid collection container has reached or exceeded a predetermined volume. In some embodiments, the volume of the fluid in the fluid collection container may be monitored by a sensor.

In some embodiments, the fluid collection pad may be removed from a pocket in the cushion, disconnected from the fluid collection system via a coupling on the tube, and disposed. A new fluid collection pad may then be coupled to the fluid collection system and restored to the pocket within the cushion. In some embodiments, a cushion cover having an opening may include the fluid collection pad within the cushion cover for discretion.

It should be appreciated that the acts of the method 200 described above are for illustrative purposes. For example, the acts of the method 200 can be performed in different orders, split into multiple acts, modified, supplemented, or combined. In an embodiment, one or more of the acts of the method 200 can be omitted from the method 200. Any of the acts of the method 200 can include using any of the portable fluid collection systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

While various aspects and embodiments have been disclosed herein, other aspects and embodiments are contemplated. The various aspects and embodiments disclosed herein are for purposes of illustration and are not intended to be limiting.

The following clauses define particular aspects and embodiments of the disclosure.
1. A fluid collection system, comprising:
   a fluid collection device configured to collect fluid discharged from a user;
   a fluid collection container configured to receive the fluid from the fluid collection device;
   a means for sensing at least one of a property or status of the fluid collection system; and
   a controller operatively coupled to the means for sensing, the controller configured to communicate at least one of the property or the status of the fluid collection system to a computing device.
2. The fluid collection system of clause 1, further comprising:
   a tube in fluid communication with the fluid collection device; and
   a pump in fluid communication with the fluid collection device and the fluid collection container, wherein the pump is configured to pull an at least partial vacuum to draw the fluid from the fluid collection device through the tube into the fluid collection container.
3. The fluid collection system of clauses 1 or 2, wherein the means for sensing includes a flow meter coupled to the tube, the flow meter being configured to detect a vacuum status or measure a volumetric flow rate of the fluid.
4. The fluid collection system of clause 3, wherein the flow meter includes an in-line ball style sight flow indicator disposed within the tube, wherein a ball float is visible when the vacuum is drawn and not visible when the vacuum is absent.
5. The fluid collection system of clause 3, wherein the flow meter includes a spring and piston flow indicator disposed within the tube, wherein a calibrated spring holds the piston such that the piston is visible when the vacuum is drawn and not visible when the vacuum is absent.
6. The fluid collection system of clause 1, wherein the fluid collection container includes a generally rigid exterior housing and an interior portion configured to contain the fluid.
7. The fluid collection system of clause 6, wherein the means for sensing includes a sensor disposed between the exterior housing and the interior portion, wherein the sensor is configured to detect a property related to the volume or mass of the fluid in the fluid collection container.
8. The fluid collection system of clause 7, wherein the interior portion is expandable and the sensor is configured to activate when the interior portion expands to a predetermined volume.
9. The fluid collection system of clause 7, wherein the interior portion is generally rigid and the sensor includes at least one of a pressure sensor, a capacitive sensor, or an optical sensor.
10. The fluid collection system of clause 1, wherein the means for sensing includes a sensor configured to detect when the fluid collection device is decoupled from the fluid collection container.
11. The fluid collection system of clause 2, further comprising a battery operably coupled to the pump, wherein the controller is configured to communicate a battery status.
12. The fluid collection system of clause 1, wherein the controller is configured to operate the pump according to an input from the sensor.
13. The fluid collection system of any of clauses 1-12, wherein the computing device includes a module attached to an arm of a wheelchair or a smartphone.
14. The fluid collection system of clause 13, wherein the computing device is wirelessly coupled to the controller.
15. The fluid collection system of any of clauses 1-14, wherein the computing device includes a programmable timer, an alert or alarm, or a display configured to display the at least one of the property or the status of the fluid collection system.
16. The fluid collection system of any of clauses 1-14, wherein the computing device is configured to provide the at least one of the property of the fluid collection system or the fluid collection system status in real-time as communicated by the controller.
17. A method of using a fluid collection system, the method comprising:
   placing a fluid collection device at least proximate to a urethra of a user;
   receiving fluid discharged from the user in a fluid collection device;
   receiving fluid discharged from the fluid collection device in a fluid collection container;
   detecting, with a means for sensing, at least one of a property or status of the fluid collection system; and
   transmitting, from a controller coupled to the means for sensing, to a computing device the at least one of the property or the status of the fluid collection system.
18. The method of clause 17, further comprising using a pump to pull a vacuum on a tube in fluid communication with the fluid collection device effective to draw fluid from the fluid collection device and into the fluid collection container.
19. The method of clause 17 or 18, wherein detecting with a means for sensing at least one of a property or status of the fluid collection system includes detecting at least one of a volume of fluid within the fluid collection device or the fluid collection container, a flow property of the fluid passing through a tube, a pump status, or a battery status.
20. The method of any of clauses 17-19, wherein transmitting, from a controller coupled to the means for sensing, to a computing device includes transmitting to a module attached to an arm of a wheelchair or a smartphone.
21. The method of any of clauses 17-20, further comprising providing, with the computing device, the at least one property or status of the fluid collection system in real-time as communicated by the controller.

## Claims

1. A fluid collection system (100), comprising:
a fluid collection device (102) configured to collect fluid discharged from a user;
a fluid collection container (106) configured to receive the fluid from the fluid collection device (102), wherein the fluid collection container (106) includes a generally rigid exterior housing (130) and a generally rigid interior portion (132) configured to contain the fluid; and
one or more sensors configured to sense at least one of a property or a status of the fluid collection system, the one or more sensors including at least a capacitive sensor (128) disposed between the exterior housing (130) and the interior portion (132) on an interior surface of the exterior housing (130), the capacitive sensor (128) configured to activate when the interior portion (132) contains a predetermined volume of fluid.

2. The fluid collection system (100) of claim 1, further comprising:
a tube (104) in fluid communication with the fluid collection device (102); and
a pump (108) in fluid communication with the fluid collection device (102) and the fluid collection container (106), wherein the pump (108) is configured to pull an at least partial vacuum to draw the fluid from the fluid collection device (102) through the tube (104) into the fluid collection container (106).

3. The fluid collection system (100) of claims 1 or 2, wherein the one or more sensors further includes a flow meter (116) coupled to the tube (104), the flow meter (116) being configured to detect a vacuum status or measure a volumetric flow rate of the fluid.

4. The fluid collection system (100) of claim 3, wherein the flow meter (116) includes an in-line ball style sight flow indicator disposed within the tube (104), wherein a ball float is visible when the vacuum is drawn and not visible when the vacuum is absent.

5. The fluid collection system (100) of claim 3, wherein the flow meter (116) includes a spring and piston flow indicator disposed within the tube (104), wherein a calibrated spring (126) holds the piston (120) such that the piston (120) is visible when the vacuum is drawn and not visible when the vacuum is absent.

6. The fluid collection system (100) of claim 1, wherein the one or more sensors further include a sensor configured to detect when the fluid collection device (102) is decoupled from the fluid collection container (106).

7. The fluid collection system (100) of claim 2, further comprising a battery (118) operably coupled to the pump (108), wherein the controller (112) is configured to communicate a battery status.

8. The fluid collection system (100) of claim 1, further comprising a controller (112) operatively coupled to the sensor (128), the controller (112) configured to coordinate display of at least one of the property or the status of the fluid collection system (100).

9. The fluid collection system (100) of claim 8, wherein the controller (112) is configured to operate the pump (108) according to an input from the sensor (128).

10. The fluid collection system (100) of either claim 8 or claim 9, wherein the controller (112) coordinates display of at least one of the property or the status of the fluid collection system (100) on a module attached to an arm of a wheelchair or a smartphone.

11. The fluid collection system (100) of any of claims 8-10, wherein the controller (112) is configured to coordinate a programmable timer (158), an alert or alarm, or a display configured to display the at least one of the property or the status of the fluid collection system (100).

12. The fluid collection system (100) of any of claims 8-11, wherein the controller (112) is configured to coordinate display of the at least one of the property of the fluid collection system (100) or the fluid collection system status in real-time.

13. A method of using a fluid collection system (100), the method comprising:
placing a fluid collection device (102) at least proximate to a urethra of a user;
receiving fluid discharged from the user in a fluid collection device (102);
receiving fluid discharged from the fluid collection device (102) in a fluid collection container (106), wherein the fluid collection container (106) includes a generally rigid exterior housing (130) and a generally rigid interior portion (132) configured to contain the fluid; and
detecting, with one or more sensors, at least one of a property or status of the fluid collection system (100), wherein the one or more sensors include at least a capacitive sensor (128) disposed between the exterior housing (130) and the interior portion (132) on an interior surface of the exterior housing (130), the capacitive sensor (128) activating when the interior portion (132) contains a predetermined volume of fluid.

14. The method of claim 13, further comprising coordinating display, with a controller (112) coupled to the one or more sensors, the at least one of the property or the status of the fluid collection system (100).

15. The method of claim 14, further comprising coordinating display, with the controller (112), of the at least one property or status of the fluid collection system (100) in real-time.
